# EUROPEAN PATENT APPLICATION

(11) **EP 1 361 278 A2**
(43) Date of publication of application: **12.11.2003**
(21) Application number: 03009512.9
(22) Date of filing: 28.04.2003
(51) Int. Cl.: C12P 13/04, C12N 15/61, C07K 14/34

(54) **Process for the production of amino acids using phosphoglucose isomerases from coryneform bacteria**

(30) Priority: 08.05.2002 DE 10220574
(71) Applicant: Degussa AG, 40474 Düsseldorf (DE)
(72) Inventor: Bathe, Brigitte, Dr., 33154 Salzkotten (DE); Marx, Achim, Dr., 97404 Banska Bystrica (SK); Pfefferle, Walter, Dr., 33790 Halle (DE); Thierbach, Georg, Dr., 33613 Bielefeld (DE)

(57) **Abstract**

The invention relates to proteins having phosphoglucose isomerase activity from coryneform bacteria, to coryneform bacteria containing such proteins, and to processes for the production of L-amino acids, especially L-lysine, using such proteins.

SEQUENCE LISTING

<110>  Degussa AG

<120>  Process for the production of amino acids with coryneform bacteria
       using phosphoglucose isomerases from coryneform bacteria.

<130>  020233 BT

<160>  6

<170>  PatentIn version 3.1

<210>  1
<211>  1758
<212>  DNA
<213>  Corynebacterium glutamicum

<220>
<221>  CDS
<222>  (1)..(1755)
<223>  pgi

<400>  1
atg gcc acg tcg aaa agc agc cca ata aac gca cct aaa ttt gtc gtg      48
Met Ala Thr Ser Lys Ser Ser Pro Ile Asn Ala Pro Lys Phe Val Val
1               5                   10                  15

ttt ccc act ttg aac act ctt cga tgc gct tgg cca caa aag caa gct      96
Phe Pro Thr Leu Asn Thr Leu Arg Cys Ala Trp Pro Gln Lys Gln Ala
            20                  25                  30

aac ctg aag atg tta ttt aac gac aat aaa gga gtt ttc atg gcg gac     144
Asn Leu Lys Met Leu Phe Asn Asp Asn Lys Gly Val Phe Met Ala Asp
        35                  40                  45

att tcg acc acc cag gtt tgg caa gac ctg acc gat cat tac tca aac     192
Ile Ser Thr Thr Gln Val Trp Gln Asp Leu Thr Asp His Tyr Ser Asn
    50                  55                  60

ttc cag gca acc act ctg cgt gaa ctt ttc aag gaa gaa aac cgc gcc     240
Phe Gln Ala Thr Thr Leu Arg Glu Leu Phe Lys Glu Glu Asn Arg Ala
65                  70                  75                  80

gag aag tac acc ttc tcc gcg gct ggc ctc cac gtc gac ctg tcg aag     288
Glu Lys Tyr Thr Phe Ser Ala Ala Gly Leu His Val Asp Leu Ser Lys
                85                  90                  95

aat ctg ctt gac gac gcc acc ctc acc aag ctc ctt gca ctg acc gaa     336
Asn Leu Leu Asp Asp Ala Thr Leu Thr Lys Leu Leu Ala Leu Thr Glu
            100                 105                 110

gaa tct ggc ctt cgc gaa cgc att gac gcg atg ttt gcc ggt gaa cac     384
Glu Ser Gly Leu Arg Glu Arg Ile Asp Ala Met Phe Ala Gly Glu His
        115                 120                 125

ctc aac aac acc gaa gac cgc gct gtc ctc cac acc gcg ctg cgc ctt     432
Leu Asn Asn Thr Glu Asp Arg Ala Val Leu His Thr Ala Leu Arg Leu
    130                 135                 140

cct gcc gaa gct gat ctg tca gta gat ggc caa gat gtt gct gct gat 480
Pro Ala Glu Ala Asp Leu Ser Val Asp Gly Gln Asp Val Ala Ala Asp
145                 150                 155                 160

gtc cac gaa gtt ttg gga cgc atg cgt gac ttc gct act gcg ctg cgc 528
Val His Glu Val Leu Gly Arg Met Arg Asp Phe Ala Thr Ala Leu Arg
                165                 170                 175

tca ggc aac tgg ttg gga cac acc ggc cac acg atc aag aag atc gtc 576
Ser Gly Asn Trp Leu Gly His Thr Gly His Thr Ile Lys Lys Ile Val
            180                 185                 190

aac att ggt atc ggt ggc tct gac ctc gga cca gcc atg gct acg aag 624
Asn Ile Gly Ile Gly Gly Ser Asp Leu Gly Pro Ala Met Ala Thr Lys
        195                 200                 205

gct ctg cgt gca tac gcg acc gct ggt atc tca gca gaa ttc gtc tcc 672
Ala Leu Arg Ala Tyr Ala Thr Ala Gly Ile Ser Ala Glu Phe Val Ser
    210                 215                 220

aac gtc gac cca gca gac ctc gtt tct gtg ttg gaa gac ctc gat gca 720
Asn Val Asp Pro Ala Asp Leu Val Ser Val Leu Glu Asp Leu Asp Ala
225                 230                 235                 240

gaa tcc aca ttg ttc gtg atc gct tcg aaa act ttc acc acc cag gag 768
Glu Ser Thr Leu Phe Val Ile Ala Ser Lys Thr Phe Thr Thr Gln Glu
                245                 250                 255

acg ctg tcc aac gct cgt gca gct cgt gct tgg ctg gta gag aag ctc 816
Thr Leu Ser Asn Ala Arg Ala Ala Arg Ala Trp Leu Val Glu Lys Leu
            260                 265                 270

ggt gaa gag gct gtc gcg aag cac ttc gtc gca gtg tcc acc aat gct 864
Gly Glu Glu Ala Val Ala Lys His Phe Val Ala Val Ser Thr Asn Ala
        275                 280                 285

gaa aag gtc gca gag ttc ggt atc gac acg gac aac atg ttc ggc ttc 912
Glu Lys Val Ala Glu Phe Gly Ile Asp Thr Asp Asn Met Phe Gly Phe
    290                 295                 300

tgg gac tgg gtc gga ggt cgt tac tcc gtg gac tcc gca gtt ggt ctt 960
Trp Asp Trp Val Gly Gly Arg Tyr Ser Val Asp Ser Ala Val Gly Leu
305                 310                 315                 320

tcc ctc atg gca gtg atc ggc cct cgc gac ttc atg cgt ttc ctc ggt 1008
Ser Leu Met Ala Val Ile Gly Pro Arg Asp Phe Met Arg Phe Leu Gly
                325                 330                 335

gga ttc cac gcg atg gat gaa cac ttc cgc acc acc aag ttc gaa gag 1056
Gly Phe His Ala Met Asp Glu His Phe Arg Thr Thr Lys Phe Glu Glu
            340                 345                 350

aac gtt cca atc ttg atg gct ctg ctc ggt gtc tgg tac tcc gat ttc 1104
Asn Val Pro Ile Leu Met Ala Leu Leu Gly Val Trp Tyr Ser Asp Phe
        355                 360                 365

tat ggt gca gaa acc cac gct gtc cta cct tat tcc gag gat ctc agc 1152
Tyr Gly Ala Glu Thr His Ala Val Leu Pro Tyr Ser Glu Asp Leu Ser
    370                 375                 380

cgt ttt gct gct tac ctc cag cag ctg acc atg gaa tca aat ggc aag     1200
Arg Phe Ala Ala Tyr Leu Gln Gln Leu Thr Met Glu Ser Asn Gly Lys
385                 390                 395                 400

tca gtc cac cgc gac ggc tcc cct gtt tcc act ggc act ggc gaa att     1248
Ser Val His Arg Asp Gly Ser Pro Val Ser Thr Gly Thr Gly Glu Ile
                405                 410                 415

tac tgg ggt gag cct ggc aca aat ggc cag cac gct ttc ttc cag ctg     1296
Tyr Trp Gly Glu Pro Gly Thr Asn Gly Gln His Ala Phe Phe Gln Leu
            420                 425                 430

atc cac cag ggc act cgc ctt gtt cca gct gat ttc att ggt ttc gct     1344
Ile His Gln Gly Thr Arg Leu Val Pro Ala Asp Phe Ile Gly Phe Ala
        435                 440                 445

cgt cca aag cag gat ctt cct gcc ggt gag cgc acc atg cat gac ctt     1392
Arg Pro Lys Gln Asp Leu Pro Ala Gly Glu Arg Thr Met His Asp Leu
    450                 455                 460

ttg atg agc aac ttc ttc gca cag acc aag gtt ttg gct ttc ggt aag     1440
Leu Met Ser Asn Phe Phe Ala Gln Thr Lys Val Leu Ala Phe Gly Lys
465                 470                 475                 480

aac gct gaa gag atc gct gcg gaa ggt gtc gca cct gag ctg gtc aac     1488
Asn Ala Glu Glu Ile Ala Ala Glu Gly Val Ala Pro Glu Leu Val Asn
                485                 490                 495

cac aag gtc atg cca ggt aat cgc cca acc acc acc att ttg gcg gag     1536
His Lys Val Met Pro Gly Asn Arg Pro Thr Thr Thr Ile Leu Ala Glu
            500                 505                 510

gaa ctt acc cct tct att ctc ggt gcg ttg atc gct ttg tac gaa cac     1584
Glu Leu Thr Pro Ser Ile Leu Gly Ala Leu Ile Ala Leu Tyr Glu His
        515                 520                 525

atc gtg atg gtt cag ggc gtg att tgg gac atc aac tcc ttc gac caa     1632
Ile Val Met Val Gln Gly Val Ile Trp Asp Ile Asn Ser Phe Asp Gln
    530                 535                 540

tgg ggt gtt gaa ctg ggc aaa cag cag gca aat gac ctc gct ccg gct     1680
Trp Gly Val Glu Leu Gly Lys Gln Gln Ala Asn Asp Leu Ala Pro Ala
545                 550                 555                 560

gtc tct ggt gaa gag gat gtt gac tcg gga gat tct tcc act gat tca     1728
Val Ser Gly Glu Glu Asp Val Asp Ser Gly Asp Ser Ser Thr Asp Ser
                565                 570                 575

ctg att aag tgg tac cgc gca aat agg tag                             1758
Leu Ile Lys Trp Tyr Arg Ala Asn Arg
            580                 585

<210>  2
<211>  585
<212>  PRT
<213>  Corynebacterium glutamicum

<400>  2
Met Ala Thr Ser Lys Ser Ser Pro Ile Asn Ala Pro Lys Phe Val Val
1               5                   10                  15

Phe Pro Thr Leu Asn Thr Leu Arg Cys Ala Trp Pro Gln Lys Gln Ala
            20                  25                  30

Asn Leu Lys Met Leu Phe Asn Asp Asn Lys Gly Val Phe Met Ala Asp
        35                  40                  45

Ile Ser Thr Thr Gln Val Trp Gln Asp Leu Thr Asp His Tyr Ser Asn
    50                  55                  60

Phe Gln Ala Thr Thr Leu Arg Glu Leu Phe Lys Glu Glu Asn Arg Ala
65                  70                  75                  80

Glu Lys Tyr Thr Phe Ser Ala Ala Gly Leu His Val Asp Leu Ser Lys
                85                  90                  95

Asn Leu Leu Asp Asp Ala Thr Leu Thr Lys Leu Leu Ala Leu Thr Glu
            100                 105                 110

Glu Ser Gly Leu Arg Glu Arg Ile Asp Ala Met Phe Ala Gly Glu His
        115                 120                 125

Leu Asn Asn Thr Glu Asp Arg Ala Val Leu His Thr Ala Leu Arg Leu
    130                 135                 140

Pro Ala Glu Ala Asp Leu Ser Val Asp Gly Gln Asp Val Ala Ala Asp
145                 150                 155                 160

Val His Glu Val Leu Gly Arg Met Arg Asp Phe Ala Thr Ala Leu Arg
                165                 170                 175

Ser Gly Asn Trp Leu Gly His Thr Gly His Thr Ile Lys Lys Ile Val
            180                 185                 190

Asn Ile Gly Ile Gly Gly Ser Asp Leu Gly Pro Ala Met Ala Thr Lys
        195                 200                 205

Ala Leu Arg Ala Tyr Ala Thr Ala Gly Ile Ser Ala Glu Phe Val Ser
    210                 215                 220

Asn Val Asp Pro Ala Asp Leu Val Ser Val Leu Glu Asp Leu Asp Ala
225                 230                 235                 240

Glu Ser Thr Leu Phe Val Ile Ala Ser Lys Thr Phe Thr Thr Gln Glu
                245                 250                 255

Thr Leu Ser Asn Ala Arg Ala Ala Arg Ala Trp Leu Val Glu Lys Leu
            260                 265                 270

Gly Glu Glu Ala Val Ala Lys His Phe Val Ala Val Ser Thr Asn Ala
        275                 280                 285

Glu Lys Val Ala Glu Phe Gly Ile Asp Thr Asp Asn Met Phe Gly Phe
    290                 295                 300

Trp Asp Trp Val Gly Gly Arg Tyr Ser Val Asp Ser Ala Val Gly Leu
305                 310                 315                 320

Ser Leu Met Ala Val Ile Gly Pro Arg Asp Phe Met Arg Phe Leu Gly
                325                 330                 335

Gly Phe His Ala Met Asp Glu His Phe Arg Thr Thr Lys Phe Glu Glu
            340                 345                 350

Asn Val Pro Ile Leu Met Ala Leu Leu Gly Val Trp Tyr Ser Asp Phe
        355                 360                 365

Tyr Gly Ala Glu Thr His Ala Val Leu Pro Tyr Ser Glu Asp Leu Ser
    370                 375                 380

Arg Phe Ala Ala Tyr Leu Gln Gln Leu Thr Met Glu Ser Asn Gly Lys
385                 390                 395                 400

Ser Val His Arg Asp Gly Ser Pro Val Ser Thr Gly Thr Gly Glu Ile
                405                 410                 415

Tyr Trp Gly Glu Pro Gly Thr Asn Gly Gln His Ala Phe Phe Gln Leu
            420                 425                 430

Ile His Gln Gly Thr Arg Leu Val Pro Ala Asp Phe Ile Gly Phe Ala
        435                 440                 445

Arg Pro Lys Gln Asp Leu Pro Ala Gly Glu Arg Thr Met His Asp Leu
    450                 455                 460

Leu Met Ser Asn Phe Phe Ala Gln Thr Lys Val Leu Ala Phe Gly Lys
465                 470                 475                 480

Asn Ala Glu Glu Ile Ala Ala Glu Gly Val Ala Pro Glu Leu Val Asn
                485                 490                 495

His Lys Val Met Pro Gly Asn Arg Pro Thr Thr Thr Ile Leu Ala Glu
            500                 505                 510

Glu Leu Thr Pro Ser Ile Leu Gly Ala Leu Ile Ala Leu Tyr Glu His
        515                 520                 525

Ile Val Met Val Gln Gly Val Ile Trp Asp Ile Asn Ser Phe Asp Gln
    530                 535                 540

Trp Gly Val Glu Leu Gly Lys Gln Gln Ala Asn Asp Leu Ala Pro Ala
545                 550                 555                 560

Val Ser Gly Glu Glu Asp Val Asp Ser Gly Asp Ser Ser Thr Asp Ser
                565                 570                 575

Leu Ile Lys Trp Tyr Arg Ala Asn Arg
            580                 585

<210> 3
<211> 1758
<212> DNA
<213> Corynebacterium glutamicum

<220>
<221> CDS
<222> (1)..(1755)
<223> pgi

<400> 3
atg gcc acg tcg aaa agc agc cca ata aac gca cct aaa ttt gtc gtg 48
Met Ala Thr Ser Lys Ser Ser Pro Ile Asn Ala Pro Lys Phe Val Val
1               5                   10                  15

ttt ccc act ttg aac act ctt cga tgc gct tgg cca caa aag caa gct 96
Phe Pro Thr Leu Asn Thr Leu Arg Cys Ala Trp Pro Gln Lys Gln Ala
            20                  25                  30

aac ctg aag atg tta ttt aac gac aat aaa gga gtt ttc atg gcg gac 144
Asn Leu Lys Met Leu Phe Asn Asp Asn Lys Gly Val Phe Met Ala Asp
        35                  40                  45

att tcg acc acc cag gct tgg caa gac ctg acc gat cat tac tca aac 192
Ile Ser Thr Thr Gln Ala Trp Gln Asp Leu Thr Asp His Tyr Ser Asn
    50                  55                  60

ttc cag gca acc act ctg cgt gaa ctt ttc aag gaa gaa aac cgc gcc 240
Phe Gln Ala Thr Thr Leu Arg Glu Leu Phe Lys Glu Glu Asn Arg Ala
65                  70                  75                  80

gag aag tac acc ttc tcc gcg gct ggc ctc cac gtc gac ctg tcg aag 288
Glu Lys Tyr Thr Phe Ser Ala Ala Gly Leu His Val Asp Leu Ser Lys
                85                  90                  95

aat ctg ctt gac gac gcc acc ctc acc aag ctc ctt gca ctg acc gaa 336
Asn Leu Leu Asp Asp Ala Thr Leu Thr Lys Leu Leu Ala Leu Thr Glu
            100                 105                 110

gaa tct ggc ctt cgc gaa cgc att gac gcg atg ttt gcc ggt gaa cac 384
Glu Ser Gly Leu Arg Glu Arg Ile Asp Ala Met Phe Ala Gly Glu His
        115                 120                 125

ctc aac aac acc gaa gac cgc gct gtc ctc cac acc gcg ctg cgc ctt 432
Leu Asn Asn Thr Glu Asp Arg Ala Val Leu His Thr Ala Leu Arg Leu
    130                 135                 140

cct ccc gaa gct gat ctg tca gta gat ggc caa gat gtt gct gct gat 480
Pro Pro Glu Ala Asp Leu Ser Val Asp Gly Gln Asp Val Ala Ala Asp
145                 150                 155                 160

gtc cac gaa gtt ttg gga cgc atg cgt gac ttc gct act gcg ctg cgc 528
Val His Glu Val Leu Gly Arg Met Arg Asp Phe Ala Thr Ala Leu Arg
                165                 170                 175

tca ggc aac tgg ttg gga cac acc ggc cac acg atc aag aag atc gtc 576
Ser Gly Asn Trp Leu Gly His Thr Gly His Thr Ile Lys Lys Ile Val
            180                 185                 190

aac att ggt atc ggt ggc tct gac ctc gga cca gcc atg gct acg aag 624
Asn Ile Gly Ile Gly Gly Ser Asp Leu Gly Pro Ala Met Ala Thr Lys
        195                 200                 205

gct ctg cgt gca tac gcg acc gct ggt atc tca gca gaa ttc gtc tcc 672
Ala Leu Arg Ala Tyr Ala Thr Ala Gly Ile Ser Ala Glu Phe Val Ser
    210                 215                 220

aac gtc gac cca gca gac ctc gtt tct gtg ttg gaa gac ctc gat gca 720
Asn Val Asp Pro Ala Asp Leu Val Ser Val Leu Glu Asp Leu Asp Ala
225                 230                 235                 240

gaa tcc aca ttg ttc gtg atc gct tcg aaa act ttt acc acc cag gag 768
Glu Ser Thr Leu Phe Val Ile Ala Ser Lys Thr Phe Thr Thr Gln Glu
                245                 250                 255

acg ctg tct aac gct cgt gca gct cgt gct tgg ctg gta gag aag ctc 816
Thr Leu Ser Asn Ala Arg Ala Ala Arg Ala Trp Leu Val Glu Lys Leu
            260                 265                 270

ggt gaa gag gct gtc gcg aag cat ttc gtc gca gtg tcc acc aat gct 864
Gly Glu Glu Ala Val Ala Lys His Phe Val Ala Val Ser Thr Asn Ala
        275                 280                 285

gaa aag gtc gca gag ttc ggt atc gac acg gac aac atg ttc ggc ttc 912
Glu Lys Val Ala Glu Phe Gly Ile Asp Thr Asp Asn Met Phe Gly Phe
    290                 295                 300

tgg gac tgg gtc gga ggt cgt tac tcc gtg gac tcc gca gtt ggt ctt 960
Trp Asp Trp Val Gly Gly Arg Tyr Ser Val Asp Ser Ala Val Gly Leu
305                 310                 315                 320

tcc ctc atg gca gtg atc ggc cct cgc gac ttc atg cgt ttc ctc ggt 1008
Ser Leu Met Ala Val Ile Gly Pro Arg Asp Phe Met Arg Phe Leu Gly
                325                 330                 335

gga ttc cac gcg atg gat gaa cac ttc cgc acc acc aag ttc gaa gag 1056
Gly Phe His Ala Met Asp Glu His Phe Arg Thr Thr Lys Phe Glu Glu
            340                 345                 350

aac gtt cca atc ttg atg gct ctg ctc ggt gtc tgg tac tcc gat ttc 1104
Asn Val Pro Ile Leu Met Ala Leu Leu Gly Val Trp Tyr Ser Asp Phe
        355                 360                 365

tat ggt gca gaa acc cac gct gtc cta cct tat tcc gag gat ctc agc 1152
Tyr Gly Ala Glu Thr His Ala Val Leu Pro Tyr Ser Glu Asp Leu Ser
    370                 375                 380

cgt ttt gct gct tac ctc cag cag ctg acc atg gaa tca aac ggc aag 1200
Arg Phe Ala Ala Tyr Leu Gln Gln Leu Thr Met Glu Ser Asn Gly Lys
385                 390                 395                 400

tca gtc cac cgc gac ggc tcc cct gtt tcc act ggc act ggc gaa att 1248
Ser Val His Arg Asp Gly Ser Pro Val Ser Thr Gly Thr Gly Glu Ile
                405                 410                 415

tac tgg ggt gag cct ggc aca aat ggc cag cac gct ttc ttc cag ctg 1296
Tyr Trp Gly Glu Pro Gly Thr Asn Gly Gln His Ala Phe Phe Gln Leu
            420                 425                 430

atc cac cag ggc act cgc ctt gtt cca gct gat ttc att ggt ttc gct 1344
Ile His Gln Gly Thr Arg Leu Val Pro Ala Asp Phe Ile Gly Phe Ala
        435                 440                 445

cgt cca aag cag gat ctt cct gcc ggt gag cgc acc atg cat gac ctt 1392
Arg Pro Lys Gln Asp Leu Pro Ala Gly Glu Arg Thr Met His Asp Leu
    450                 455                 460

ttg atg agc aac ttc ttc gca cag acc aag gtt ttg gct ttc ggt aag 1440
Leu Met Ser Asn Phe Phe Ala Gln Thr Lys Val Leu Ala Phe Gly Lys
465                 470                 475                 480

aac gct gaa gag atc gct gcg gaa ggt gtc gca cct gag ctg gtc aac 1488
Asn Ala Glu Glu Ile Ala Ala Glu Gly Val Ala Pro Glu Leu Val Asn
                485                 490                 495

cac aag gtc atg cca ggt aat cgc cca acc acc acc att ttg gcg gag 1536
His Lys Val Met Pro Gly Asn Arg Pro Thr Thr Thr Ile Leu Ala Glu
            500                 505                 510

gaa ctt acc cct tct att ctc ggt gcg ttg atc gct ttg tac gaa cac 1584
Glu Leu Thr Pro Ser Ile Leu Gly Ala Leu Ile Ala Leu Tyr Glu His
        515                 520                 525

atc gtg atg gtt cag ggc gtg att tgg gac atc aac tcc ttc gac caa 1632
Ile Val Met Val Gln Gly Val Ile Trp Asp Ile Asn Ser Phe Asp Gln
    530                 535                 540

tgg ggt gtt gaa ctg ggc aaa cag cag gca aat gac ctc gct ccg gct 1680
Trp Gly Val Glu Leu Gly Lys Gln Gln Ala Asn Asp Leu Ala Pro Ala
545                 550                 555                 560

gtc tct ggt gaa gag gat gtt gac tcg gga gat tct tcc act gat tca 1728
Val Ser Gly Glu Glu Asp Val Asp Ser Gly Asp Ser Ser Thr Asp Ser
                565                 570                 575

ctg att aag tgg tac cgc gca aat agg tag 1758
Leu Ile Lys Trp Tyr Arg Ala Asn Arg
            580                 585

<210> 4
<211> 585
<212> PRT
<213> Corynebacterium glutamicum

<400> 4
Met Ala Thr Ser Lys Ser Ser Pro Ile Asn Ala Pro Lys Phe Val Val
1               5                   10                  15

Phe Pro Thr Leu Asn Thr Leu Arg Cys Ala Trp Pro Gln Lys Gln Ala
            20                  25                  30

Asn Leu Lys Met Leu Phe Asn Asp Asn Lys Gly Val Phe Met Ala Asp
        35                  40                  45

Ile Ser Thr Thr Gln Ala Trp Gln Asp Leu Thr Asp His Tyr Ser Asn
    50                  55                  60

Phe Gln Ala Thr Thr Leu Arg Glu Leu Phe Lys Glu Glu Asn Arg Ala
65                  70                  75                  80

Glu Lys Tyr Thr Phe Ser Ala Ala Gly Leu His Val Asp Leu Ser Lys
                85                  90                  95

Asn Leu Leu Asp Asp Ala Thr Leu Thr Lys Leu Leu Ala Leu Thr Glu
            100                 105                 110

Glu Ser Gly Leu Arg Glu Arg Ile Asp Ala Met Phe Ala Gly Glu His
        115                 120                 125

Leu Asn Asn Thr Glu Asp Arg Ala Val Leu His Thr Ala Leu Arg Leu
    130                 135                 140

Pro Pro Glu Ala Asp Leu Ser Val Asp Gly Gln Asp Val Ala Ala Asp
145                 150                 155                 160

Val His Glu Val Leu Gly Arg Met Arg Asp Phe Ala Thr Ala Leu Arg
                165                 170                 175

Ser Gly Asn Trp Leu Gly His Thr Gly His Thr Ile Lys Lys Ile Val
            180                 185                 190
Asn Ile Gly Ile Gly Gly Ser Asp Leu Gly Pro Ala Met Ala Thr Lys
        195                 200                 205
Ala Leu Arg Ala Tyr Ala Thr Ala Gly Ile Ser Ala Glu Phe Val Ser
    210                 215                 220
Asn Val Asp Pro Ala Asp Leu Val Ser Val Leu Glu Asp Leu Asp Ala
225                 230                 235                 240
Glu Ser Thr Leu Phe Val Ile Ala Ser Lys Thr Phe Thr Thr Gln Glu
                245                 250                 255
Thr Leu Ser Asn Ala Arg Ala Ala Arg Ala Trp Leu Val Glu Lys Leu
            260                 265                 270
Gly Glu Glu Ala Val Ala Lys His Phe Val Ala Val Ser Thr Asn Ala
        275                 280                 285
Glu Lys Val Ala Glu Phe Gly Ile Asp Thr Asp Asn Met Phe Gly Phe
    290                 295                 300
Trp Asp Trp Val Gly Gly Arg Tyr Ser Val Asp Ser Ala Val Gly Leu
305                 310                 315                 320
Ser Leu Met Ala Val Ile Gly Pro Arg Asp Phe Met Arg Phe Leu Gly
                325                 330                 335
Gly Phe His Ala Met Asp Glu His Phe Arg Thr Thr Lys Phe Glu Glu
            340                 345                 350
Asn Val Pro Ile Leu Met Ala Leu Leu Gly Val Trp Tyr Ser Asp Phe
        355                 360                 365
Tyr Gly Ala Glu Thr His Ala Val Leu Pro Tyr Ser Glu Asp Leu Ser
    370                 375                 380
Arg Phe Ala Ala Tyr Leu Gln Gln Leu Thr Met Glu Ser Asn Gly Lys
385                 390                 395                 400
Ser Val His Arg Asp Gly Ser Pro Val Ser Thr Gly Thr Gly Glu Ile
                405                 410                 415
Tyr Trp Gly Glu Pro Gly Thr Asn Gly Gln His Ala Phe Phe Gln Leu
            420                 425                 430
Ile His Gln Gly Thr Arg Leu Val Pro Ala Asp Phe Ile Gly Phe Ala
        435                 440                 445
Arg Pro Lys Gln Asp Leu Pro Ala Gly Glu Arg Thr Met His Asp Leu
    450                 455                 460
Leu Met Ser Asn Phe Phe Ala Gln Thr Lys Val Leu Ala Phe Gly Lys
465                 470                 475                 480
Asn Ala Glu Glu Ile Ala Ala Glu Gly Val Ala Pro Glu Leu Val Asn
                485                 490                 495

His Lys Val Met Pro Gly Asn Arg Pro Thr Thr Thr Ile Leu Ala Glu
            500                 505                 510

Glu Leu Thr Pro Ser Ile Leu Gly Ala Leu Ile Ala Leu Tyr Glu His
        515                 520                 525

Ile Val Met Val Gln Gly Val Ile Trp Asp Ile Asn Ser Phe Asp Gln
    530                 535                 540

Trp Gly Val Glu Leu Gly Lys Gln Gln Ala Asn Asp Leu Ala Pro Ala
545                 550                 555                 560

Val Ser Gly Glu Glu Asp Val Asp Ser Gly Asp Ser Ser Thr Asp Ser
                565                 570                 575

Leu Ile Lys Trp Tyr Arg Ala Asn Arg
            580                 585

<210>  5
<211>  1758
<212>  DNA
<213>  Corynebacterium glutamicum

<220>
<221>  CDS
<222>  (1)..(1755)
<223>  pgi

<400>  5
atg gcc acg tcg aaa agc agc cca ata aac gca cct aaa ttt gtc gtg        48
Met Ala Thr Ser Lys Ser Ser Pro Ile Asn Ala Pro Lys Phe Val Val
1               5                   10                  15

ttt ccc act ttg aac act ctt cga tgc gct tgg cca caa aag caa gct        96
Phe Pro Thr Leu Asn Thr Leu Arg Cys Ala Trp Pro Gln Lys Gln Ala
            20                  25                  30

aac ctg aag atg tta ttt aac gac aat aaa gga gtt ttc atg gcg gac       144
Asn Leu Lys Met Leu Phe Asn Asp Asn Lys Gly Val Phe Met Ala Asp
        35                  40                  45

att tcg acc acc cag gtt tgg caa gac ctg acc gat cat tac tca aac       192
Ile Ser Thr Thr Gln Val Trp Gln Asp Leu Thr Asp His Tyr Ser Asn
    50                  55                  60

ttc cag gca acc act ctg cgt gaa ctt ttc aag gaa gaa aac cgc gcc       240
Phe Gln Ala Thr Thr Leu Arg Glu Leu Phe Lys Glu Glu Asn Arg Ala
65                  70                  75                  80

gag aag tac acc ttc tcc gcg gct ggc ctc cac gtc gac ctg tcg aag       288
Glu Lys Tyr Thr Phe Ser Ala Ala Gly Leu His Val Asp Leu Ser Lys
                85                  90                  95

aat ctg ctt gac gac gcc acc ctc acc aag ctc ctt gca ctg acc gaa       336
Asn Leu Leu Asp Asp Ala Thr Leu Thr Lys Leu Leu Ala Leu Thr Glu
            100                 105                 110

gaa tct ggc ctt cgc gaa cgc att gac gcg atg ttt gcc ggt gaa cac       384
Glu Ser Gly Leu Arg Glu Arg Ile Asp Ala Met Phe Ala Gly Glu His
        115                 120                 125

ctc aac aac acc gaa gac cgc gct gtc ctc cac acc gcg ctg cgc ctt 432
Leu Asn Asn Thr Glu Asp Arg Ala Val Leu His Thr Ala Leu Arg Leu
    130                 135                 140

cct gcc gaa gct gat ctg tca gta gat ggc caa gat gtt gct gct gat 480
Pro Ala Glu Ala Asp Leu Ser Val Asp Gly Gln Asp Val Ala Ala Asp
145                 150                 155                 160

gtc cac gaa gtt ttg gga cgc atg cgt gac ttc gct act gcg ctg cgc 528
Val His Glu Val Leu Gly Arg Met Arg Asp Phe Ala Thr Ala Leu Arg
                165                 170                 175

tca ggc aac tgg ttg gga cac acc ggc cac acg atc aag aag atc gtc 576
Ser Gly Asn Trp Leu Gly His Thr Gly His Thr Ile Lys Lys Ile Val
            180                 185                 190

aac att ggt atc ggt ggc tct gac ctc gga cca gcc atg gct acg aag 624
Asn Ile Gly Ile Gly Gly Ser Asp Leu Gly Pro Ala Met Ala Thr Lys
        195                 200                 205

gct ctg cgt gca tac gcg acc gct ggt atc tca gca gaa ttc gtc tcc 672
Ala Leu Arg Ala Tyr Ala Thr Ala Gly Ile Ser Ala Glu Phe Val Ser
    210                 215                 220

aac gtc gac cca gca gac ctc gtt tct gtg ttg gaa gac ctc gat gca 720
Asn Val Asp Pro Ala Asp Leu Val Ser Val Leu Glu Asp Leu Asp Ala
225                 230                 235                 240

gaa tcc aca ttg ttc gtg atc gct tcg aaa act ttc acc acc cag gag 768
Glu Ser Thr Leu Phe Val Ile Ala Ser Lys Thr Phe Thr Thr Gln Glu
                245                 250                 255

acg ctg tcc aac gct cgt gca gct cgt gct tgg ctg gta gag aag ctc 816
Thr Leu Ser Asn Ala Arg Ala Ala Arg Ala Trp Leu Val Glu Lys Leu
            260                 265                 270

ggt gaa gag gct gtc gcg aag cac ttc gtc gca gtg tcc acc aat gct 864
Gly Glu Glu Ala Val Ala Lys His Phe Val Ala Val Ser Thr Asn Ala
        275                 280                 285

gaa aag gtc gca gag ttc ggt atc gac acg gac aac atg ttc ggc ttc 912
Glu Lys Val Ala Glu Phe Gly Ile Asp Thr Asp Asn Met Phe Gly Phe
    290                 295                 300

tgg gac tgg gtc gga ggt cgt tac tcc gtg gac tcc gca gtt ggt ctt 960
Trp Asp Trp Val Gly Gly Arg Tyr Ser Val Asp Ser Ala Val Gly Leu
305                 310                 315                 320

tcc ctc atg gca gtg atc ggc cct cgc gac ttc atg cgt ttc ctc ggt 1008
Ser Leu Met Ala Val Ile Gly Pro Arg Asp Phe Met Arg Phe Leu Gly
                325                 330                 335

gga ttc cac gcg atg gat gaa cac ttc cgc acc acc aag ttc gaa gag 1056
Gly Phe His Ala Met Asp Glu His Phe Arg Thr Thr Lys Phe Glu Glu
            340                 345                 350

aac gtt cca atc ttg atg gct ctg ctc ggt gtc tgg tac tcc gat ttc 1104
Asn Val Pro Ile Leu Met Ala Leu Leu Gly Val Trp Tyr Ser Asp Phe
        355                 360                 365

tat ggt gca gaa acc cac gct gtc cta cct tat tcc gag gat ctc agc 1152
Tyr Gly Ala Glu Thr His Ala Val Leu Pro Tyr Ser Glu Asp Leu Ser
    370                 375                 380

cgt ttt gct gct tac ctc cag cag ctg acc atg gag acc aat ggc aag 1200
Arg Phe Ala Ala Tyr Leu Gln Gln Leu Thr Met Glu Thr Asn Gly Lys
385                 390                 395                 400

tca gtc cac cgc gac ggc tcc cct gtt tcc act ggc act ggc gaa att 1248
Ser Val His Arg Asp Gly Ser Pro Val Ser Thr Gly Thr Gly Glu Ile
                405                 410                 415

tac tgg ggt gag cct ggc aca aat ggc cag cac gct ttc ttc cag ctg 1296
Tyr Trp Gly Glu Pro Gly Thr Asn Gly Gln His Ala Phe Phe Gln Leu
            420                 425                 430

atc cac cag ggc act cgc ctt gtt cca gct gat ttc att ggt ttc gct 1344
Ile His Gln Gly Thr Arg Leu Val Pro Ala Asp Phe Ile Gly Phe Ala
        435                 440                 445

cgt cca aag cag gat ctt cct gcc ggt gag cgc acc atg cat gac ctt 1392
Arg Pro Lys Gln Asp Leu Pro Ala Gly Glu Arg Thr Met His Asp Leu
    450                 455                 460

ttg atg agc aac ttc ttc gca cag acc aag gtt ttg gct ttc ggt aag 1440
Leu Met Ser Asn Phe Phe Ala Gln Thr Lys Val Leu Ala Phe Gly Lys
465                 470                 475                 480

aac gct gaa gag atc gct gcg gaa ggt gtc gca cct gag ctg gtc aac 1488
Asn Ala Glu Glu Ile Ala Ala Glu Gly Val Ala Pro Glu Leu Val Asn
                485                 490                 495

cac aag gtc gtg cca ggt aat cgc cca acc acc acc att ttg gcg gag 1536
His Lys Val Val Pro Gly Asn Arg Pro Thr Thr Thr Ile Leu Ala Glu
            500                 505                 510

gaa ctt acc cct tct att ctc ggt gcg ttg atc gct ttg tac gaa cac 1584
Glu Leu Thr Pro Ser Ile Leu Gly Ala Leu Ile Ala Leu Tyr Glu His
        515                 520                 525

acc gtg atg gtt cag ggc gtg att tgg gac atc aac tcc ttc gac caa 1632
Thr Val Met Val Gln Gly Val Ile Trp Asp Ile Asn Ser Phe Asp Gln
    530                 535                 540

tgg ggt gtt gaa ctg ggc aaa cag cag gca aat gac ctc gct ccg gct 1680
Trp Gly Val Glu Leu Gly Lys Gln Gln Ala Asn Asp Leu Ala Pro Ala
545                 550                 555                 560

gtc tct ggt gaa gag gat gtt gac tcg gga gat tct tcc act gat tca 1728
Val Ser Gly Glu Glu Asp Val Asp Ser Gly Asp Ser Ser Thr Asp Ser
                565                 570                 575

ctg att aag tgg tac cgc gca aat agg tag 1758
Leu Ile Lys Trp Tyr Arg Ala Asn Arg
            580                 585

<210> 6
<211> 585
<212> PRT
<213> Corynebacterium glutamicum

<400> 6

Met Ala Thr Ser Lys Ser Ser Pro Ile Asn Ala Pro Lys Phe Val Val
1               5                   10                  15

Phe Pro Thr Leu Asn Thr Leu Arg Cys Ala Trp Pro Gln Lys Gln Ala
            20                  25                  30

Asn Leu Lys Met Leu Phe Asn Asp Asn Lys Gly Val Phe Met Ala Asp
        35                  40                  45

Ile Ser Thr Thr Gln Val Trp Gln Asp Leu Thr Asp His Tyr Ser Asn
    50                  55                  60

Phe Gln Ala Thr Thr Leu Arg Glu Leu Phe Lys Glu Glu Asn Arg Ala
65                  70                  75                  80

Glu Lys Tyr Thr Phe Ser Ala Ala Gly Leu His Val Asp Leu Ser Lys
                85                  90                  95

Asn Leu Leu Asp Asp Ala Thr Leu Thr Lys Leu Leu Ala Leu Thr Glu
            100                 105                 110

Glu Ser Gly Leu Arg Glu Arg Ile Asp Ala Met Phe Ala Gly Glu His
        115                 120                 125

Leu Asn Asn Thr Glu Asp Arg Ala Val Leu His Thr Ala Leu Arg Leu
    130                 135                 140

Pro Ala Glu Ala Asp Leu Ser Val Asp Gly Gln Asp Val Ala Ala Asp
145                 150                 155                 160

Val His Glu Val Leu Gly Arg Met Arg Asp Phe Ala Thr Ala Leu Arg
                165                 170                 175

Ser Gly Asn Trp Leu Gly His Thr Gly His Thr Ile Lys Lys Ile Val
            180                 185                 190

Asn Ile Gly Ile Gly Gly Ser Asp Leu Gly Pro Ala Met Ala Thr Lys
        195                 200                 205

Ala Leu Arg Ala Tyr Ala Thr Ala Gly Ile Ser Ala Glu Phe Val Ser
    210                 215                 220

Asn Val Asp Pro Ala Asp Leu Val Ser Val Leu Glu Asp Leu Asp Ala
225                 230                 235                 240

Glu Ser Thr Leu Phe Val Ile Ala Ser Lys Thr Phe Thr Thr Gln Glu
                245                 250                 255

Thr Leu Ser Asn Ala Arg Ala Ala Arg Ala Trp Leu Val Glu Lys Leu
            260                 265                 270

Gly Glu Glu Ala Val Ala Lys His Phe Val Ala Val Ser Thr Asn Ala
        275                 280                 285

Glu Lys Val Ala Glu Phe Gly Ile Asp Thr Asp Asn Met Phe Gly Phe
    290                 295                 300

Trp Asp Trp Val Gly Gly Arg Tyr Ser Val Asp Ser Ala Val Gly Leu
305                 310                 315                 320

Ser Leu Met Ala Val Ile Gly Pro Arg Asp Phe Met Arg Phe Leu Gly
                325                 330                 335

Gly Phe His Ala Met Asp Glu His Phe Arg Thr Thr Lys Phe Glu Glu
            340                 345                 350

Asn Val Pro Ile Leu Met Ala Leu Leu Gly Val Trp Tyr Ser Asp Phe
        355                 360                 365

Tyr Gly Ala Glu Thr His Ala Val Leu Pro Tyr Ser Glu Asp Leu Ser
    370                 375                 380

Arg Phe Ala Ala Tyr Leu Gln Gln Leu Thr Met Glu Thr Asn Gly Lys
385                 390                 395                 400

Ser Val His Arg Asp Gly Ser Pro Val Ser Thr Gly Thr Gly Glu Ile
                405                 410                 415

Tyr Trp Gly Glu Pro Gly Thr Asn Gly Gln His Ala Phe Phe Gln Leu
            420                 425                 430

Ile His Gln Gly Thr Arg Leu Val Pro Ala Asp Phe Ile Gly Phe Ala
        435                 440                 445

Arg Pro Lys Gln Asp Leu Pro Ala Gly Glu Arg Thr Met His Asp Leu
    450                 455                 460

Leu Met Ser Asn Phe Phe Ala Gln Thr Lys Val Leu Ala Phe Gly Lys
465                 470                 475                 480

Asn Ala Glu Glu Ile Ala Ala Glu Gly Val Ala Pro Glu Leu Val Asn
                485                 490                 495

His Lys Val Val Pro Gly Asn Arg Pro Thr Thr Thr Ile Leu Ala Glu
            500                 505                 510

Glu Leu Thr Pro Ser Ile Leu Gly Ala Leu Ile Ala Leu Tyr Glu His
        515                 520                 525

Thr Val Met Val Gln Gly Val Ile Trp Asp Ile Asn Ser Phe Asp Gln
    530                 535                 540

Trp Gly Val Glu Leu Gly Lys Gln Gln Ala Asn Asp Leu Ala Pro Ala
545                 550                 555                 560

Val Ser Gly Glu Glu Asp Val Asp Ser Gly Asp Ser Ser Thr Asp Ser
                565                 570                 575

Leu Ile Lys Trp Tyr Arg Ala Asn Arg
            580                 585

## Description

### Field of the invention

The invention relates to proteins having phosphoglucose isomerase activity from coryneform bacteria, to coryneform bacteria containing such proteins, and to a process for the production of L-amino acids, especially L-lysine, using such proteins.

### Prior art

L-amino acids, especially L-lysine, are used in human medicine and in the pharmaceuticals industry, in the foodstuffs industry and, very especially, in the feeding of animals.

It is known that amino acids are produced by fermentation of strains of coryneform bacteria, especially Corynebacterium glutamicum. Because of their great importance, attempts are continuously being made to improve the production processes. Improvements to the processes may concern measures relating to the fermentation, such as, for example, stirring and oxygen supply, or the composition of the nutrient media, such as, for example, the sugar concentration during the fermentation, or working up to the product form by, for example, ion-exchange chromatography, or the intrinsic performance properties of the microorganism itself.

In order to improve the performance properties of such microorganisms, methods of mutagenesis, selection and mutant selection are employed. Such methods yield strains which are resistant to antimetabolites, such as, for example, the lysine analogue S-(2-aminoethyl)-cysteine, or are auxotrophic for metabolites that are important in terms of regulation, and which produce L-amino acids.

For a number of years, methods of recombinant DNA technology have also been used for improving the strain of L-amino acid-producing strains of Corynebacterium glutamicum, by amplifying or attenuating individual amino acid biosynthesis genes and studying the effect on L-amino acid production.

The nucleotide sequence of the chromosome of Corynebacterium glutamicum belongs to the prior art and has been published, for example, within the scope of EP-A-1108790 and WO 01/00844.

Studies relating to the pgi gene and the enzyme phosphoglucose isomerase coded for by that gene are described in EP-A-1087015 and in WO 01/07626.

### Object of the invention

The object of the invention is to provide bases for improved processes for the production by fermentation of L-amino acids, especially L-lysine, with coryneform bacteria.

### Summary of the invention

The invention provides proteins having phosphoglucose isomerase activity from coryneform bacteria, especially Corynebacterium glutamicum, characterised by a length of 585 amino acid residues, the N-terminus optionally being shortened by 1 or 2, from 3 to 17, from 18 to 21, from 22 to 36 or from 37 to 46 amino acid residues, and nucleotide sequences that code for the mentioned proteins.

The invention also provides proteins having phosphoglucose isomerase activity from coryneform bacteria, especially Corynebacterium glutamicum, characterised by a length of 585 amino acid residues, the N-terminus optionally being shortened by 1 or 2, from 3 to 17, from 18 to 21, from 22 to 36 or from 37 to 46 amino acid residues and the C-terminus having an amino acid sequence selected from the group SEQ ID NO:2 corresponding to position 47 to 585, SEQ ID NO:4 corresponding to position 47 to 585 and SEQ ID NO:6 corresponding to position 47 to 585, and nucleotide sequences that code for the mentioned proteins.

Finally, the invention provides proteins having phosphoglucose isomerase activity from coryneform bacteria, especially Corynebacterium glutamicum having an amino acid sequence selected from the group SEQ ID NO:2, SEQ ID NO:2 corresponding to position 2 to 585, SEQ ID NO:4, SEQ ID NO:4 corresponding to position 2 to 585, SEQ ID NO:6 and SEQ ID NO:6 corresponding to position 2 to 585, and nucleotide sequences from coryneform bacteria that code for the mentioned proteins shown in SEQ ID NO:1, SEQ ID NO:3 and SEQ ID NO:5, including the variants arising from the degeneracy of the genetic code.

The expression "from coryneform bacteria" means that the corresponding proteins or nucleic acids come from coryneform bacteria or have their origin therein.

Nucleotide sequences that code for the proteins according to the invention having phosphoglucose isomerase activity can also be designated pgi genes or alleles.

The invention also provides bacteria, such as, for example, Corynebacterium glutamicum or Escherichia coli, which contain the nucleotide sequences, or pgi genes or alleles, according to the invention.

Likewise, vectors containing nucleotide sequences according to the invention are claimed.

The invention also provides coryneform bacteria in which proteins having phosphoglucose isomerase activity are present in enhanced or attenuated form, the mentioned proteins being characterised by a length of 585 amino acid residues, and the N-terminus of the mentioned proteins optionally being shortened by 1 or 2, from 3 to 17, from 18 to 21, from 22 to 36 or from 37 to 46 amino acid residues.

The term "enhancement" or "enhance" in this connection describes the increase in the intracellular activity or concentration of one or more enzymes or proteins in a microorganism that are coded for by the corresponding DNA, by, for example, increasing the number of copies of the gene or genes, using a strong promoter or using a gene or allele that codes for a corresponding enzyme or protein having a high level of activity, and optionally by combining those measures.

By the measures of enhancement, especially overexpression, the activity or concentration of the corresponding protein is generally increased by at least 10%, 25%, 50%, 75%, 100%, 150%, 200%, 300%, 400% or 500%, at the maximum up to 1000% or 2000%, based on that of the wild-type protein or the activity or concentration of the protein in the starting microorganism.

The term "attenuation" or "attenuate" in this context describes the diminution or exclusion of the intracellular activity or concentration of one or more enzymes or proteins in a microorganism that are coded for by the corresponding DNA, by, for example, using a weak promoter or using a gene or allele that codes for a corresponding enzyme having a low level of activity, or by inactivating the corresponding enzyme or protein or gene, and optionally by combining those measures.

By the measures of attenuation, the activity or concentration of the corresponding protein is generally lowered to from 0 to 75%, from 0 to 50%, from 0 to 25%, from 0 to 10% or from 0 to 5% of the activity or concentration of the wild-type protein, or of the activity or concentration of the protein in the starting microorganism.

### Detailed Description of the Invention

Where L-amino acids or amino acids are mentioned hereinbelow, they are to be understood as being one or more amino acids, including their salts, selected from the group L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine.

Finally, the invention also provides a process for the production of one or more L-amino acids, especially selected from the group L-lysine, L-valine, L-threonine and L-methionine, with coryneform bacteria, which process is characterised by the following steps:
a) enhancement of proteins having phosphoglucose isomerase activity in the coryneform bacteria,
b) fermentation of the bacteria obtained in step a),
c) concentration of the amino acids in the medium, or in the fermentation liquor, or in the cells of the bacteria, and
d) isolation of the amino acids, constituents of the fermentation liquor and/or the biomass in totality or in part (from ≥ 0 to 100%) optionally remaining in the product,
the mentioned proteins being characterised by a length of 585 amino acid residues, and the N-terminus of the mentioned proteins optionally being shortened by 1 or 2, from 3 to 17, from 18 to 21, from 22 to 36 or from 37 to 46 amino acid residues.

Enhancement of the mentioned proteins having phosphoglucose isomerase activity is preferably used when the amino acid to be produced has a high NADPH consumption in the scope of its biosynthesis. That is the case, for example, with the amino acids L-lysine, L-valine, L-threonine and L-methionine.

The invention further provides a process for the production of one or more L-amino acids, especially selected from the group L-tryptophan, L-phenylalanine and L-tyrosine, with coryneform bacteria, which process is characterised by the following steps:
a) attenuation of proteins having phosphoglucose isomerase activity in the coryneform bacteria,
b) fermentation of the bacteria obtained in step a),
c) concentration of the amino acids in the medium, or in the fermentation liquor, or in the cells of the bacteria, and
d) isolation of the amino acids, constituents of the fermentation liquor and/or the biomass in totality or in part (from ≥ 0 to 100%) optionally remaining in the product,
the mentioned proteins being characterised by a length of 585 amino acid residues, and the N-terminus of the mentioned proteins optionally being shortened by 1 or 2, from 3 to 17, from 18 to 21, from 22 to 36 or from 37 to 46 amino acid residues.

Attenuation of the mentioned proteins having phosphoglucose isomerase activity is preferably used when a metabolite of the pentose phosphate cycle is a precursor of the amino acid to be produced. Erythrose-4-phosphate, for example, is a metabolite of the pentose phosphate cycle and a precursor of the aromatic amino acids L-tryptophan, L-phenylalanine and L-tyrosine. Summaries of the metabolic reactions and metabolites of the pentose phosphate cycle are to be found in textbooks of microbiology and biochemistry, such as, for example, the textbook of G. Gottschalk "Bacterial Metabolism" (2nd ed., Springer-Verlag, New York, USA, 1986).

The coryneform bacteria used preferably already produce L-amino acids before the enhancement or attenuation of the proteins according to the invention having phosphoglucose isomerase activity.

The microorganisms provided by the present invention are able to produce L-amino acids from glucose, saccharose, lactose, fructose, maltose, molasses, starch, cellulose or from glycerol or ethanol or from acetic acid or lactic acid. They may be representatives of coryneform bacteria especially of the genus Corynebacterium. In the case of the genus Corynebacterium, special mention may be made of the species Corynebacterium glutamicum, which is known to those skilled in the art for its ability to produce L-amino acids.

Suitable strains of the genus Corynebacterium, especially of the species Corynebacterium glutamicum, are especially the known wild-type strains
Corynebacterium glutamicum ATCC13032,
Corynebacterium acetoglutamicum ATCC15806,
Corynebacterium acetoacidophilum ATCC13870,
Corynebacterium melassecola ATCC17965,
Corynebacterium thermoaminogenes FERM BP-1539,
Corynebacterium efficiens DSM44549
Brevibacterium flavum ATCC14067,
Brevibacterium lactofermentum ATCC13869 and
Brevibacterium divaricatum ATCC14020
and L-amino acid-producing mutants or strains prepared therefrom

such as, for example, the L-lysine-producing strains
Corynebacterium glutamicum FERM-P 1709,
Brevibacterium flavum FERM-P 1708,
Brevibacterium lactofermentum FERM-P 1712,
Corynebacterium glutamicum FERM-P 6463,
Corynebacterium glutamicum FERM-P 6464,
Corynebacterium glutamicum DSM 5715,
Corynebacterium glutamicum DM58-1 and
Corynebacterium glutamicum DSM12866
or such as, for example, the L-tryptophan-producing strains
Corynebacterium glutamicum ATCC21850 and
Corynebacterium glutamicum KY9218(pKW9901).

Strains designated "ATCC" can be obtained from the American Type Culture Collection (Manassas, VA, USA). Strains designated "FERM" can be obtained from the National Institute of Advanced Industrial Science and Technology (AIST Tsukuba Central 6, 1-1-1 Higashi, Tsukuba Ibaraki, Japan). Strains designated "DSM" can be obtained from the Deutsche Sammlung von Mikroorganismen und Zellkulturen (DSMZ, Braunschweig, Germany). The strain Corynebacterium glutamicum DM58-1 is described in EP-A-0358940. The strain Corynebacterium glutamicum KY9218 (pKW9901) is described in Ikeda *et al.* (Bioscience Biotechnology and Biochemistry 58 (4), 674-678 (1994)).

During work on the present invention, it was possible to identify proteins having phosphoglucose isomerase activity in Corynebacterium glutamicum, which are shown in SEQ ID NO:2, SEQ ID NO:4 and SEQ ID NO:6. N-terminal amino acids can be cleaved by enzymes proper to the host, so-called peptidases. A known enzyme is aminopeptidase, which cleaves N-terminal methionine.

For determining the N-terminal amino acid sequence of a protein such as phosphoglucose isomerase, the "His-tag" method can be used. In that method, the coding region of the corresponding gene is lengthened at the 3' end generally by from 1 to 10, typically 6, histidine codons. To that end, the coding region is inserted into corresponding vectors, as are described in the prior art, for example in the Qiagen Product Guide 2002 from Qiagen GmbH (Hilden, Germany). It is also possible to prepare the corresponding gene by means of the polymerase chain reaction using oligonucleotide primers which contain the histidine codons, and then insert the gene lengthened by the histidine codons into a suitable vector. Expression of the protein preferably takes place in Escherichia coli or Corynebacterium glutamicum. The protein provided with histidine labelling is then purified from the crude extract by affinity chromatography and the N-terminus is determined by Edman degradation. Purification of the protein may also be carried out by two-dimensional gel chromatography.

It has also been found that an improvement in the lysine production of lysine-producing coryneform bacteria can be achieved when the proteins according to the invention having phosphoglucose isomerase activity are enhanced.

In order to achieve an enhancement, either the expression or the catalytic properties of the proteins according to the invention can be increased. The two measures are optionally combined.

In order to achieve an overexpression, the number of copies of the corresponding genes can be increased, or the promoter and regulation region or the ribosome binding site, which is located upstream of the structural gene, can be mutated. Expression cassettes inserted upstream of the structural gene have a similar effect. By means of inducible promoters it is additionally possible to increase the expression in the course of the production of amino acids by fermentation. Expression is also improved by measures to prolong the life of the m-RNA. Furthermore, the enzyme activity is also enhanced by preventing degradation of the enzyme protein. The genes or gene constructs may either be present in plasmids with different numbers of copies or be integrated and amplified in the chromosome. Alternatively, overexpression of the genes in question may also be achieved by changing the composition of the medium and the manner in which culturing is carried out.

The person skilled in the art will find instructions thereon *inter alia* in Martin *et al*. (Bio/Technology 5, 137-146 (1987)), in Guerrero *et al*. (Gene 138, 35-41 (1994)), Tsuchiya and Morinaga (Bio/Technology 6, 428-430 (1988)), in Eikmanns *et al.* (Gene 102, 93-98 (1991)), in European patent specification 0 472 869, in US patent 4,601,893, in Schwarzer and Pühler (Bio/Technology 9, 84-87 (1991), in Reinscheid *et al.* (Applied and Environmental Microbiology 60, 126-132 (1994)), in LaBarre *et al.* (Journal of Bacteriology 175, 1001-1007 (1993)), in patent application WO 96/15246, in Malumbres *et al.* (Gene 134, 15-24 (1993)), in Jensen and Hammer (Biotechnology and Bioengineering 58, 191-195 (1998)), in Makrides (Microbiological Reviews 60:512-538 (1996)) and in known textbooks of genetics and molecular biology.

A common method of achieving an overexpression consists in the use of episomal plasmids. Suitable plasmids are those which are replicated in coryneform bacteria. Many known plasmid vectors, such as, for example, pZ1 (Menkel *et al.,* Applied and Environmental Microbiology (1989) 64: 549-554), pEKEx1 (Eikmanns *et al*., Gene 102:93-98 (1991)) or pHS2-1 (Sonnen *et al.,* Gene 107:69-74 (1991)), are based on the cryptic plasmids pHM1519, pBL1 or pGA1. Other plasmid vectors, such as, for example, those which are based on pCG4 (US-A 4,489,160) or pNG2 (Serwold-Davis *et al.,* FEMS Microbiology Letters 66, 119-124 (1990)) or pAG1 (US-A 5,158,891), may likewise be used.

Also suitable are those plasmid vectors with the aid of which the process of gene amplification by integration into the chromosome can be applied, as has been described, for example, by Reinscheid *et al.* (Applied and Environmental Microbiology 60, 126-132 (1994)) for the duplication or amplification of the hom-thrB operon. In that method, the complete gene is cloned into a plasmid vector that is able to replicate in a host (typically E. coli), but not in C. glutamicum. Suitable vectors are, for example, pSUP301 (Simon *et al.,* Bio/Technology 1, 784-791 (1983)), pK18mob or pK19mob (Schäfer *et al.,* Gene 145, 69-73 (1994)), pGEM-T (Promega Corporation, Madison, WI, USA), pCR2.1-TOPO (Shuman (1994). Journal of Biological Chemistry 269:32678-32684; US-A 5,487,993), pCR®Blunt (Invitrogen, Groningen, Netherlands; Bernard *et al*., Journal of Molecular Biology, 234: 534-541 (1993)), pEM1 (Schrumpf *et al*., 1991, Journal of Bacteriology 173:4510-4516) or pBGS8 (Spratt *et al*., 1986, Gene 41: 337-342). The plasmid vector containing the gene to be amplified is then transferred to the desired strain of C. glutamicum by conjugation or transformation. The method of conjugation is described, for example, in Schäfer *et al.* (Applied and Environmental Microbiology 60, 756-759 (1994)). Methods of transformation are described, for example, in Thierbach *et al*. (Applied Microbiology and Biotechnology 29, 356-362 (1988)), Dunican and Shivnan (Bio/Technology 7, 1067-1070 (1989)) and Tauch *et al*. (FEMS Microbiological Letters 123, 343-347 (1994)). After homologous recombination by means of a cross-over occurrence, the resulting strain contains at least two copies of the gene in question.

In order to achieve an attenuation, either the expression or the catalytic properties of the proteins according to the invention can be reduced. The two measures are optionally combined.

Gene expression can be diminished by carrying out the culturing in a suitable manner or by genetic alteration (mutation) of the signal structures of gene expression.

Signal structures of gene expression are, for example, repressor genes, activator genes, operators, promoters, attenuators, ribosome-binding sites, the start codon and terminators. The person skilled in the art will find information thereon, for example, in patent application WO 96/15246, in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in Pátek *et al*. (Microbiology 142: 1297 (1996)), and in known textbooks of genetics and molecular biology, such as, for example, the textbook of Knippers ("Molekulare Genetik", 6th edition, Georg Thieme Verlag, Stuttgart, Germany, 1995) or that of Winnacker ("Gene und Klone", VCH Verlagsgesellschaft, Weinheim, Germany, 1990). Methods of "anti-sense" RNA technology can also be used.

Mutations that lead to a change in or diminution of the catalytic properties of enzyme proteins are known from the prior art; examples which may be mentioned are the works of Qiu and Goodman (Journal of Biological Chemistry 272: 8611-8617 (1997)), Sugimoto *et al.* (Bioscience Biotechnology and Biochemistry 61: 1760-1762 (1997)) and Möckel ("Die Threonindehydratase aus Corynebacterium glutamicum: Aufhebung der allosterischen Regulation und Struktur des Enzyms", Berichte des Forschungszentrums Jülichs, Jül-2906, ISSN09442952, Jülich, Germany, 1994). Summaries are to be found in known textbooks of genetics and molecular biology, such as, for example, that of Hagemann ("Allgemeine Genetik", Gustav Fischer Verlag, Stuttgart, 1986).

As mutations there come into consideration transitions, transversions, insertions and deletions of at least one base pair. In dependence on the effect of the amino acid substitution on the enzyme activity, missense mutations or nonsense mutations are referred to. As a result of nonsense mutations, sense codons are converted into stop codons and the translation breaks off prematurely. Insertions or deletions of at least one base pair in a gene lead to frame shift mutations, as a result of which incorrect amino acids are incorporated or the translation breaks off prematurely. Deletions of one or more codons typically lead to complete loss of enzyme activity.

The mentioned mutations are preferably incorporated into the nucleotide sequences of the genes which code for the N-terminus of the proteins according to the invention having phosphoglucose isomerase. The N-terminus includes especially the amino acid residues 1 to 22, 1 to 37 or 1 to 46, or 3 to 22, 3 to 37 or 3 to 46, or 18 to 22, 18 to 37 or 18 to 46, or 22 to 37, 22 to 46 or 37 to 46 of SEQ ID NO:2, 4 or 6.

Instructions for the production of such mutations are part of the prior art and can be found in known textbooks of genetics and molecular biology, such as, for example, the textbook of Knippers ("Molekulare Genetik", 6th edition, Georg Thieme Verlag, Stuttgart, Germany, 1995), that of Winnacker ("Gene und Klone", VCH Verlagsgesellschaft, Weinheim, Germany, 1990) or that of Hagemann ("Allgemeine Genetik", Gustav Fischer Verlag, Stuttgart, 1986).

The invention also provides nucleotide sequences that are substantially identical with the described nucleotide sequences. They include those nucleotide sequences which contain at least one further, especially conservative, amino acid substitution.

In the case of aromatic amino acids, the expression conservative substitution is used when phenylalanine, tryptophan and tyrosine are substituted for one another. In the case of hydrophobic amino acids, the expression conservative substitution is used when leucine, isoleucine and valine are substituted for one another. In the case of polar amino acids, the expression conservative substitution is used when glutamine and asparagine are substituted for one another. In the case of basic amino acids, the expression conservative substitution is used when arginine, lysine and histidine are substituted for one another. In the case of acid amino acids, the expression conservative substitution is used when aspartic acid and glutamic acid are substituted for one another. In the case of amino acids containing hydroxyl groups, the expression conservative substitution is used when serine and threonine are substituted for one another.

A common method of mutating genes of C. glutamicum is the method of gene disruption and gene replacement described by Schwarzer and Pühler (Bio/Technology 9, 84-87 (1991)).

In the method of gene disruption, a central portion of the coding region of the gene in question is cloned into a plasmid vector which is able to replicate in a host (typically E. coli), but not in C. glutamicum. Suitable vectors are, for example, pSUP301 (Simon *et al.,* Bio/Technology 1, 784-791 (1983)), pK18mo or pK19mob (Schäfer *et al.,* Gene 145, 69-73 (1994)), pK18mobsacB or pK19mobsacB (Jäger *et al.,* Journal of Bacteriology 174: 5462-5465 (1992)), pGEM-T (Promega Corporation, Madison, WI, USA), pCR2.1-TOPO (Shuman (1994). Journal of Biological Chemistry 269:32678-32684; US Patent 5,487,993), pCR®Blunt (Invitrogen, Groningen, Netherlands; Bernard *et al*., Journal of Molecular Biology, 234: 534-541 (1993)) or pEM1 (Schrumpf *et al.,* 1991, Journal of Bacteriology 173:4510-4516). The plasmid vector containing the central portion of the coding region of the gene is then transferred to the desired strain of C. glutamicum by conjugation or transformation. The method of conjugation is described, for example, in Schäfer *et al.* (Applied and Environmental Microbiology 60, 756-759 (1994)). Methods of transformation are described, for example, in Thierbach *et* *al.* (Applied Microbiology and Biotechnology 29, 356-362 (1988)), Dunican and Shivnan (Bio/Technology 7, 1067-1070 (1989)) and Tauch *et al*. (FEMS Microbiological Letters 123, 343-347 (1994)). After homologous recombination by means of a cross-over occurrence, the coding region of the gene in question is disrupted by the vector sequence, and two incomplete alleles lacking the 3'- and the 5'-end, respectively, are obtained. That method has been used, for example, by Fitzpatrick *et al.* (Applied Microbiology and Biotechnology 42, 575-580 (1994)) to exclude the recA gene of C. glutamicum.

In the gene replacement method, a mutation, such as, for example, a deletion, insertion or base substitution, is produced *in vitro* in the gene in question. The allele that is produced is in turn cloned into a vector that is not replicative for C. glutamicum, and the latter is then transferred to the desired host of C. glutamicum by transformation or conjugation. After homologous recombination by means of a first cross-over occurrence effecting integration and by means of a suitable second cross-over occurrence effecting an excision in the target gene or in the target sequence, incorporation of the mutation or of the allele is achieved. That method has been used, for example, by Peters-Wendisch *et al.* (Microbiology 144, 915-927 (1998)) to exclude the pyc gene of C. glutamicum by means of a deletion.

In addition, it may be advantageous for the production of L-amino acids, in addition to the enhancement or attenuation of the proteins according to the invention having phosphoglucose isomerase activity or of the genes or nucleotide sequences coding therefor, to enhance, especially overexpress, one or more enzymes of the biosynthesis pathway in question, of glycolysis, of the anaplerotic pathway, of the citric acid cycle, of the pentose phosphate cycle, of amino acid export, and optionally regulatory proteins.

The use of endogenous genes or endogenous nucleotide sequences is preferred. "Endogenous genes" or "endogenous nucleotide sequences" are to be understood as meaning the genes or alleles or the nucleotide sequences present in the population of a species.

Accordingly, for the production of L-lysine, it is possible, in addition to enhancing the proteins according to the invention having phosphoglucose isomerase activity, to enhance, especially overexpress, one or more genes selected from the group
- the gene lysC coding for a feed-back resistant aspartate kinase (Accession No.P26512, EP-B-0387527; EP-A-0699759; WO 00/63388),
- the gene dapA coding for dihydrodipicolinate synthase (EP-B 0 197 335),
- the gene lysE coding for the lysine export protein (DE-A-195 48 222),
- the gene pyc coding for pyruvate carboxylase (WO 99/18228, US-A-6,171,833),
- the gene gdh coding for glutamate dehydrogenase (US-A-6,355,454),
- the gene zwf coding for glucose 6-phosphate dehydrogenase (JP-A-09224661),
- the gene mqo coding for malate:quinone oxidoreductase (Molenaar *et al*., European Journal of Biochemistry 254, 395-403 (1998)), and
- the gene zwa1 coding for the Zwa1 protein (EP-A-1111062).

It can also be advantageous for the production of L-lysine, in addition to enhancing the proteins according to the invention having phosphoglucose isomerase activity, to attenuate, especially reduce or exclude the expression of, one or more genes selected from the group
- the gene ccpA1 coding for catabolite control protein A (WO 02/18419),
- the gene pck coding for phosphoenol pyruvate carboxykinase (EP-A-1094111),
- the gene fda coding for fructose bisphosphate aldolase (Molecular Microbiology 3 (11), 1625-1637 (1989); ACCESSION Number X17313),
- the gene zwa2 coding for the Zwa2 protein (EP-A-1106693).

The microorganisms produced according to the invention also form part of the invention and can be cultivated continuously or discontinuously by the batch process or by the fed batch or repeated fed batch process for the purposes of the production of L-amino acids. A summary of known cultivation methods is described in the textbook of Chmiel (Bioprozesstechnik 1. Einführung in die Bioverfahrenstechnik (Gustav Fischer Verlag, Stuttgart, 1991)) or in the textbook of Storhas (Bioreaktoren und periphere Einrichtungen (Vieweg Verlag, Braunschweig/Wiesbaden, 1994)).

The culture medium to be used must meet the requirements of the strains in question in a suitable manner. Descriptions of culture media for various microorganisms are to be found in the handbook "Manual of Methods for General Bacteriology" of the American Society for Bacteriology (Washington D.C., USA, 1981).

There may be used as the carbon source sugars and carbohydrates, such as, for example, glucose, saccharose, lactose, fructose, maltose, molasses, starch and cellulose, oils and fats, such as, for example, soybean oil, sunflower oil, groundnut oil and coconut oil, fatty acids, such as, for example, palmitic acid, stearic acid and linoleic acid, alcohols, such as, for example, glycerol and ethanol, and organic acids, such as, for example, acetic acid or lactic acid. Those substances may be used individually or in the form of a mixture.

There may be used as the nitrogen source organic nitrogen-containing compounds, such as peptones, yeast extract, meat extract, malt extract, corn steep liquor, soybean flour and urea, or inorganic compounds, such as ammonium sulfate, ammonium chloride, ammonium phosphate, ammonium carbonate and ammonium nitrate. The nitrogen sources may be used individually or in the form of a mixture.

There may be used as the phosphorus source phosphoric acid, potassium dihydrogen phosphate or dipotassium hydrogen phosphate or the corresponding sodium-containing salts. The culture medium must also contain salts of metals, such as, for example, magnesium sulfate or iron sulfate, which are necessary for growth. Finally, essential growth substances, such as amino acids and vitamins, may be used in addition to the above-mentioned substances. Suitable precursors may also be added to the culture medium. The mentioned substances may be added to the culture in the form of a single batch, or they may be fed in in a suitable manner during the cultivation.

In order to control the pH value of the culture, basic compounds, such as sodium hydroxide, potassium hydroxide, ammonia or ammonia water, or acid compounds, such as phosphoric acid or sulfuric acid, are expediently used. In order to control the development of foam, anti-foams, such as, for example, fatty acid polyglycol esters, may be used.

In order to maintain the stability of plasmids, suitable substances having a selective action, such as, for example, antibiotics, may be added to the medium. In order to maintain aerobic conditions, oxygen or gas mixtures containing oxygen, such as, for example, air, are introduced into the culture. The temperature of the culture is normally from 20°C to 45°C and preferably from 25°C to 40°C. The culture is continued until the maximum amount of the desired product has formed. That aim is normally achieved within a period of from 10 hours to 160 hours.

Methods of determining L-amino acids are known from the prior art. The analysis may be carried out as described in Spackman *et al.* (Analytical Chemistry, 30, (1958), 1190-1206) by anion-exchange chromatography with subsequent ninhydrin derivatisation, or it may be carried out by reversed phase HPLC, as described in Lindroth *et al.* (Analytical Chemistry (1979) 51: 1167-1174).

## Claims

1. Proteins having phosphoglucose isomerase activity from coryneform bacteria, **characterised by** a length of 585 amino acid residues, the N-terminus optionally being shortened by 1 or 2, from 3 to 17, from 18 to 21, from 22 to 36 or from 37 to 46 amino acid residues.

2. Proteins having phosphoglucose isomerase activity from coryneform bacteria, **characterised by** a length of 585 amino acid residues, the N-terminus optionally being shortened by 1 or 2, from 3 to 17, from 18 to 21, from 22 to 36 or from 37 to 46 amino acid residues and the C-terminus having an amino acid sequence selected from the group SEQ ID NO:2 corresponding to position 47 to 585, SEQ ID NO:4 corresponding to position 47 to 585 and SEQ ID NO:6 corresponding to position 47 to 585.

3. Proteins having phosphoglucose isomerase activity from coryneform bacteria having an amino acid sequence selected from the group SEQ ID NO:2, SEQ ID NO:2 corresponding to position 2 to 585, SEQ ID NO:4, SEQ ID NO:4 corresponding to position 2 to 585, SEQ ID NO:6 and SEQ ID NO:6 corresponding to position 2 to 585.

4. Proteins having phosphoglucose isomerase activity from coryneform bacteria according to claim 1, 2 or 3, **characterised in that** the coryneform bacteria are Corynebacterium glutamicum.

5. Isolated nucleotide sequences that code for proteins according to claim 1, 2 or 3.

6. Coryneform bacteria in which proteins having phosphoglucose isomerase activity are present in enhanced or attenuated form, the mentioned proteins being **characterised by** a length of 585 amino acid residues, and the N-terminus of the mentioned proteins optionally being shortened by 1 or 2, from 3 to 17, from 18 to 21, from 22 to 36 or from 37 to 46 amino acid residues.

7. Coryneform bacteria according to claim 6, **characterised in that** the coryneform bacteria are Corynebacterium glutamicum.

8. Process for the production of amino acids, especially with coryneform bacteria, which process is **characterised by** the following steps:
a) enhancement of proteins having phosphoglucose isomerase activity in the coryneform bacteria,
b) fermentation of the bacteria obtained in step a),
c) concentration of the amino acids in the medium, or in the fermentation liquor, or in the cells of the bacteria, and
d) isolation of the amino acids, constituents of the fermentation liquor and/or the biomass in totality or in part (from ≥ 0 to 100%) optionally remaining in the product,
the mentioned proteins being **characterised by** a length of 585 amino acid residues, and the N-terminus of the mentioned proteins optionally being shortened by 1 or 2, from 3 to 17, from 18 to 21, from 22 to 36 or from 37 to 46 amino acid residues.

9. Process according to claim 8, **characterised in that** the coryneform bacteria are Corynebacterium glutamicum.

10. Process according to claim 8, **characterised in that** the amino acids are one or more amino acids selected from the group L-lysine, L-valine, L-threonine and L-methionine.

11. Process according to claim 8, **characterised in that** the amino acids are L-lysine.

12. Process for the production of amino acids, especially with coryneform bacteria, which process is **characterised by** the following steps:
a) attenuation of proteins having phosphoglucose isomerase activity in the coryneform bacteria,
b) fermentation of the bacteria obtained in step a),
c) concentration of the amino acids in the medium, or in the fermentation liquor, or in the cells of the bacteria, and
d) isolation of the amino acids, constituents of the fermentation liquor and/or the biomass in totality or in part (from ≥ 0 to 100%) optionally remaining in the product,
the mentioned proteins being **characterised by** a length of 585 amino acid residues, and the N-terminus of the mentioned proteins optionally being shortened by 1 or 2, from 3 to 17, from 18 to 21, from 22 to 36 or from 37 to 46 amino acid residues.

13. Process according to claim 12, **characterised in that** the coryneform bacteria are Corynebacterium glutamicum.

14. Process according to claim 13, **characterised in that** the amino acids are one or more amino acids selected from the group L-tryptophan, L-phenylalanine and L-tyrosine.

15. Vectors containing the nucleic acids according to claim 5.

16. Bacteria containing the nucleic acids according to claim 5.
